# DEMANDE DE BREVET EUROPEEN

(11) **EP 0 611 554 A1**
(43) Date de publication de la demande: **24.08.1994**
(21) Numéro de dépôt: 94420055.9
(22) Date de dépôt: 16.02.1994
(51) Int. Cl.: A61B 17/60

(54) **Prothèse intervertébrale extradiscale pour le contrôle des variations de la distance intervertébrale au moyen d'un amortisseur double**

(30) Priorité: 17.02.1993 FR 9302184; 17.05.1993 FR 9306174
(71) Demandeur: PSI, 69002 Lyon (FR)
(72) Inventeur: Navas, Fernand, F-69260 Charbonnières les Bains (FR)
(74) Mandataire: Monnier, Guy

(57) **Abrégé**

Amortisseur du genre comprenant
(1) un corps élastique dont le volume est inférieur à celui de la chambre déterminée par la position à l'état libre du piston par rapport au fond du cylindre, constitué par les éléments (1 ou 1' ou 10 ou 10')
(2) un cylindre creux (1a) percé d'une lumière débouchante (1d) et dont l'une des extrémités est fermée par un fond (1b) solidaire sur sa face externe d'un tenon (1c) tandis que l'autre extrémité reçoit un disque (2) qui permet par l'intermédiaire d'une bague d'assemblage (3) la liaison avec un autre élément (1), un manchon (4) placé dans le cylindre (1) et dont l'une des extrémités est fermée par un fond (4a) alors que l'autre reçoit une face du disque (2) sur laquelle est prévu un bossage (2a), une encoche (4b) étant ménagée sur la périphérie du manchon (4) et une vis 5 dont la tête sphérique (5a) est montée à pivotement entre le disque (2) et le fond (4a) et un corps élastique (7) placé entre le manchon (4) et le fond (2b) du cylindre (1) de manière à amortir les déplacements de celui-ci sous l'effet des déplacements de la vis, pour régler le mouvement entre deux vertèbres de manière à se rapprocher le plus possible de l'allure physiologique d'un mouvement discal.

## Description

La présente invention a trait à une prothèse à amortisseur double permettant la fonction de stabilisateur intervertébral par le biais du contrôle de la distance interpédiculaire.

On connaît des amortisseurs de ce genre qui sont généralement à simple ou double effet et qui sont susceptibles de résister progressivement de façon exponentielle à l'avance d'un piston, de telle manière qu'après une certaine course du piston, l'amortisseur s'oppose à tout déplacement du piston au-delà d'une valeur déterminée de ce déplacement. Pour cela, l'amortisseur renferme de part et d'autre du piston un corps élastique dont le volume est inférieur à celui de la chambre déterminée par la position à l'état libre du piston par rapport au fond du cylindre.

La variation du volume de la chambre ou du compartiment entraîne une déformation du corps élastique correspondant à laquelle s'oppose l'indéformabilité des parois de la chambre, de telle sorte qu'une force antagoniste s'oppose progressivement au déplacement du piston jusqu'à l'arrêter lorsque cette force devient exponentielle.

Les perfectionnements qui font l'objet de la présente invention permettent la réalisation d'un amortisseur comprenant deux éléments séparés identiques assemblés entre eux et comportant des moyens pour la régulation du mouvement de flexion extension entre deux vertèbres en développant une courbe exponentielle dans les deux directions de manière à se rapprocher le plus possible de l'allure physiologique d'un mouvement discal.

Ainsi, chaque élément comprend un cylindre creux percé d'une lumière débouchante et dont l'une des extrémités est fermée par un fond solidaire sur sa face externe d'un tenon, tandis que l'autre extrémité reçoit un disque qui permet, par l'intermédiaire d'une bague d'assemblage, la liaison avec un autre élément, un manchon placé dans le cylindre et dont l'une des extrémités est fermée par un fond alors que l'autre reçoit une face du disque sur laquelle est prévu un bossage, une encoche étant ménagée sur la périphérie du manchon, une vis dont la tête sphérique est montée à pivotement entre le disque et le fond et un corps élastique placé entre le manchon et le fond du cylindre de manière à amortir les déplacements de celui-ci sous l'effet des déplacements de la vis.

Une variante consiste en ce que pour chaque élément, il soit prévu au moins un cylindre dont l'une des extrémités est fermée par un fond, tandis que l'autre extrémité comporte une bague d'assemblage qui permet la liaison avec un autre élément, la partie médiane des cylindres étant pourvue d'un trou transversal pour la mise en place d'un manchon tubulaire prévu pour recevoir à force une sphère solidaire d'une vis pédiculaire, et en ce qu'entre chaque manchon et le fond est placé un corps élastique alors qu'un autre corps élastique est introduit entre chaque manchon et la bague d'assemblage.

De plus, chaque manchon est maintenu élastiquement dans le sens axial des cylindres, les déplacements du manchon dans un sens ou dans l'autre s'effectuant à l'encontre de la réaction du corps élastique correspondant.

Le dessin annexé, donné à titre d'exemple, permettra de mieux comprendre l'invention, les caractéristiques qu'elle présente et les avantages qu'elle est susceptible de procurer :

Fig. 1 est une vue en perspective éclatée montrant un élément de l'amortisseur suivant la présente invention.

Fig. 2 est une vue en perspective représentant l'élément monté sur lequel vient s'encliqueter la bague d'assemblage pour la liaison avec un autre élément.

Fig. 3 est une coupe représentant l'amortisseur pourvu de deux éléments de fig. 1 réunis au moyen d'une bague d'assemblage pour remplir la fonction de stabilisateur intervertébral.

Fig. 4 est une vue illustrant une première variante de l'amortisseur suivant l'invention.

Fig. 5 est une vue montrant une seconde variante de l'amortisseur pour sa mise en place sur des vis pédiculaires particulières.

Fig. 6 est une vue illustrant une troisième variante de l'amortisseur suivant l'invention.

Fig. 7 est une vue schématique représentant la mise en place de plusieurs amortisseurs montrés en fig. 6.

Fig. 8 est une vue montrant une variante d'assemblage des amortisseurs représentés en fig. 6.

Fig. 9 est une courbe représentant les variations de la force antagoniste développée par l'amortisseur.

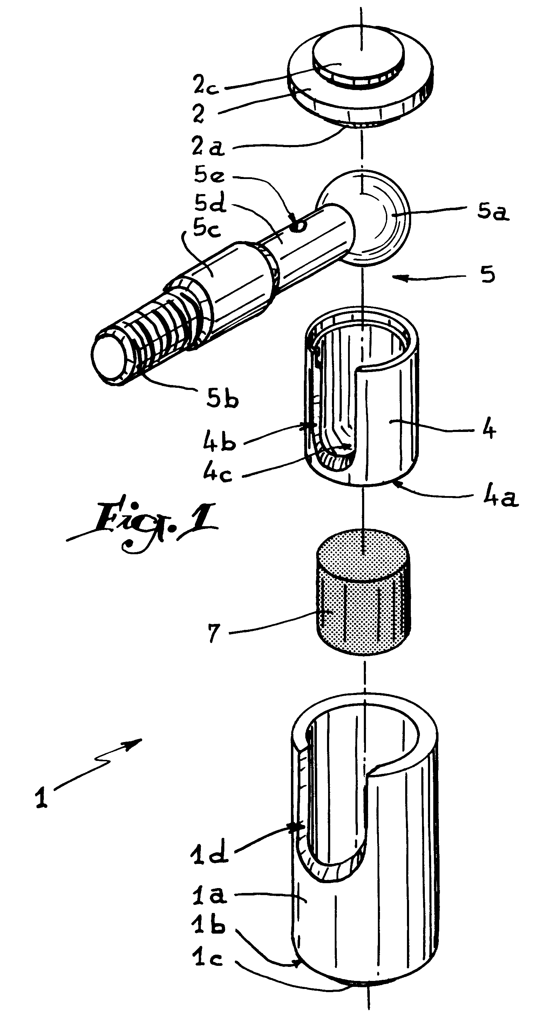

On a représenté en fig. 1 un élément 1 isolé qui doit être assemblé avec un autre identique pour constituer l'amortisseur intervertébral suivant l'invention.

Chaque élément 1 comprend un cylindre creux 1a dont l'une des extrémités est fermée par un fond 1b, tandis que l'autre, qui est prévue ouverte, est destinée à recevoir un disque 2. La face extérieure du fond 1b est usinée pour constituer un tenon en T 1c qui permet la liaison avec une bague d'assemblage 3, comme on le verra mieux plus loin.

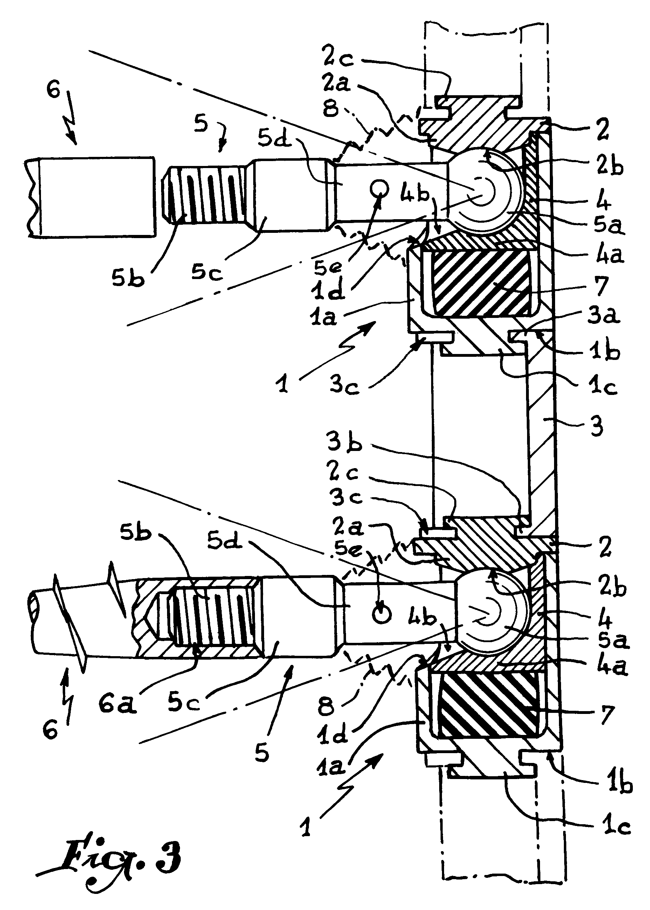

Le cylindre 1a est percé d'une lumière ou encoche longitudinale 1d qui débouche du côté de l'extrémité qui doit recevoir le disque 2. Ce dernier comprend sur l'une des faces un bossage 2a dont le milieu est usiné pour constituer une creusure 2b de forme correspondant à une partie de sphère, comme représenté en fig. 3. Le disque 2 comporte sur la face opposée à celle comprenant le bossage 2a une saillie 2c en forme de tenon identique à celui 1c prévu sur le fond 1b du cylindre 1a.

Un manchon tubulaire 4 est fermé à l'une de ses extrémités par un fond 4a, tandis que l'autre est prévue ouverte pour recevoir le bossage 2a du disque 2 lorsque l'élément 1 est monté. La périphérie du manchon 4 est pourvue d'une encoche longitudinale 4b qui débouche du côté de l'extrémité recevant le bossage 2a. La partie interne du fond 4a est usinée pour déterminer une creusure 4c de rayon identique à celle 2b prévue dans le bossage 2a du disque 2. L'élément 1 comprend encore une vis 5 dont l'une des extrémités comporte une sphère 5a dont le rayon correspond à celui des creusures. La vis 5 comporte à l'opposé de la sphère 5a une partie filetée 5b qui permet sa fixation dans une vis pédiculaire 6, comme on le verra mieux plus loin. Entre la partie filetée 5b et la sphère 5a, la vis 5 est munie d'une portée 5c et d'une partie lisse cylindrique 5d qui est percée de part en part d'un trou transversal 5e. On observe que le diamètre de la partie 5d est inférieur à celui de la portée 5c.

Entre le fond 4a du manchon 4 et le fond 1b du cylindre 1a est placé un corps élastique 7 tel qu'un bloc de caoutchouc naturel ou synthétique. Le volume à l'état libre du corps élastique 7 est légèrement inférieur à celui de la chambre dans laquelle il est placé qui est déterminé par le volume intérieur du cylindre 1a défini entre son fond 1b et le manchon 4. Le manchon 4 est introduit dans le cylindre 1a afin que son encoche 4b soit placée en face de celle 1d du cylindre. Ensuite la tête 5a de la vis 5 est introduite à l'intérieur du manchon 4 de manière que la partie lisse 5d traverse les encoches 1d et 4b.

Le disque 2 qui ferme le manchon et l'élément 1 est soudé sur la périphérie du cylindre 1a de manière que son bossage 2a soit placé à l'intérieur du manchon 4. On remarque que la creusure 2b du bossage 2a vient en appui contre la sphère 5a de la vis 5 pour la guider angulairement à l'intérieur du manchon 4.

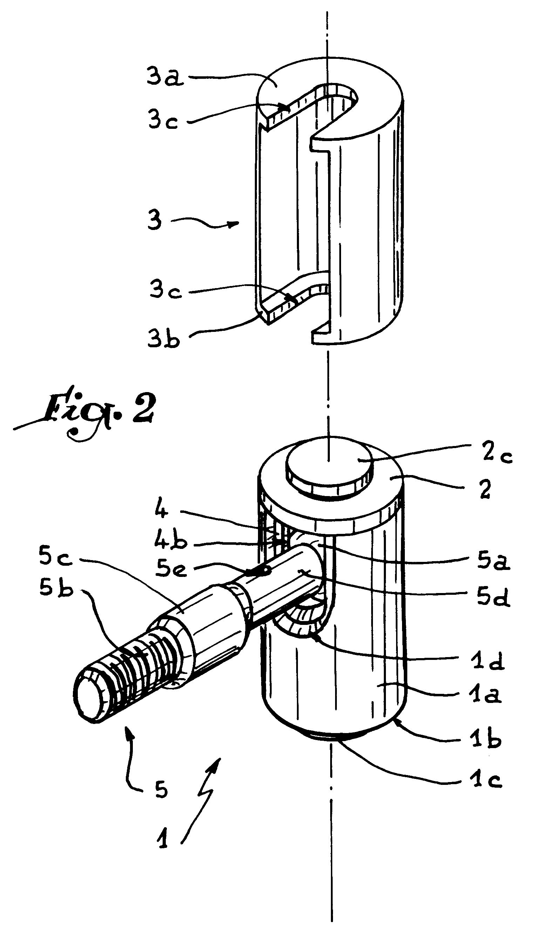

En fig. 2, on a représenté un élément 1 complètement monté sur lequel vient s'engager la bague d'assemblage 3. Cette dernière permet de réunir deux éléments 1 identiques pour constituer l'amortisseur suivant la présente invention. La bague 3 présente un profil cylindrique creux dont les extrémités sont fermées par des joues parallèles 3a et 3b qui sont chacune pourvues d'une encoche 3c dont le diamètre est légèrement inférieur à celui des tenons 1c et 2c prévus respectivement sur le cylindre 1a et sur le disque 2. La bague 3 peut être totalement rigide ou souple ou articulée de manière que les éléments 1 puissent pivoter l'un par rapport à l'autre.

En fig. 3, on a représenté deux éléments 1 décrits précédemment et assemblés l'un avec l'autre au moyen de la bague 3 en vue de constituer l'amortisseur. Chacune des vis 5 est fixée dans une vis pédiculaire 6 préalablement vissée dans le corps de deux vertèbres à stabiliser. Chaque vis pédiculaire 6 présente à l'une de ses extrémités un trou taraudé fileté 6a dans lequel est vissée la partie filetée des vis 5. Les vis 5 peuvent être également fixées par tout autre moyen à l'intérieur des vis pédiculaires 6. Autour de chaque vis 5 est prévu un soufflet 8 qui entoure la partie lisse 5d et qui vient prendre appui sur le bossage 5c et sur la face extérieure du cylindre 1a. Les soufflets sont comprimés au moment du montage de manière que les encoches 1b et 4b ne soient jamais en contact avec les parties telles que du sang ou de la chair, ce qui risquerait de bloquer les déplacements angulaires de l'amortisseur.

On remarque que les éléments 1 sont montés dans le même sens, de manière que la bague 3 coopère d'une part avec le premier élément au moyen du tenon 2c et d'autre part avec le second élément par l'intermédiaire du tenon 1c. Cette disposition particulière permet de stabiliser et d'amortir les déplacements des vertèbres soit en traction, soit en compression.

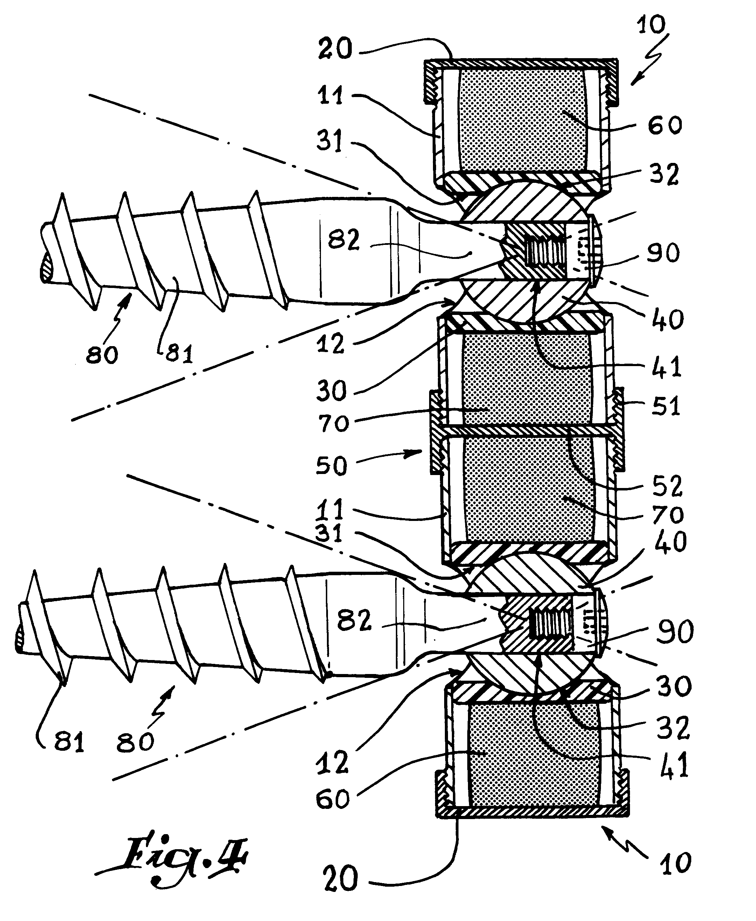

On a représenté en fig. 4 une première variante de l'amortisseur suivant l'invention qui comprend deux éléments identiques 10 assemblés entre eux.

Chaque élément comprend un cylindre 11 ouvert à ses deux extrémités et dont l'une d'entre elles est fermée par un fond 20 préférablement vissé, mais qui pourrait être fixé par tout autre moyen.

La partie médiane du cylindre 10 est pourvue d'un trou transversal traversant 12 dans lequel est engagé un manchon tubulaire 30 ou piston. Le manchon tubulaire 30 est réalisé soit en métal soit en une matière plastique appropriée telle que le polyéthylène. Il présente un alésage 31 comportant dans sa partie médiane une creusure 32 de forme correspondant à une partie de sphère. Dans cette creusure 32 est engagée une sphère 40 dont le rayon peut correspondre ou être plus évasé que celui de ladite creusure.

Les deux cylindres 11 sont assemblés au moyen d'une bague 50 constituée par une virole cylindrique 51 dont les extrémités se vissent respectivement sur les deux extrémités opposées et voisines des deux cylindres 11. On observe que la bague 50 est pourvue d'une paroi transversale médiane 52 dont l'épaisseur peut varier suivant l'utilisation de l'amortisseur et qui constitue une cloison séparatrice des cylindres 11. Cette cloison 11 peut être aussi articulée ou flexible.

Entre chaque manchon 30 et le fond 20 du cylindre correspondant est placé un corps élastique 60 tel qu'un bloc de caoutchouc naturel ou synthétique. Le volume à l'état libre du corps élastique 60 est légèrement inférieur à celui de la chambre dans lequel il est placé et qui est déterminé par le volume intérieur du cylindre 11 défini entre son fond 20 et le manchon 30. Un autre corps élastique 70 identique au corps 60 est placé entre chaque manchon 30 et la cloison séparatrice 52 de la bague d'assemblage 50.

De cette même manière, chaque manchon 30 est maintenu élastiquement dans le sens axial du cylindre 11, les déplacements du manchon dans un sens ou dans l'autre s'effectuant à l'encontre de la réaction élastique du corps correspondant.

En vue de l'application de l'amortisseur décrit ci-dessus à la stabilisation de deux vertèbres voisines d'une colonne vertébrale, on prévoit de percer dans chaque sphère 40 un trou radial 42 dans lequel est introduite une extrémité cylindrique 82 d'une vis pédiculaire 80. En effet, la vis pédiculaire 80 est ancrée dans l'apophyse d'une vertèbre par son extrémité filetée 81, tandis que son autre extrémité cylindrique 82 est associée à l'alésage 41 de la sphère 40 par l'intermédiaire d'une vis 90. La vis 90 comporte une tête qui présente un diamètre plus grand que celui de la partie cylindrique 82, de manière à constituer une butée empêchant la séparation de la sphère 40 et de la vis 80 tout en permettant à cette dernière à se déplacer longitudinalement dans l'alésage 41.

Bien entendu, c'est grâce au trou transversal 12 de chaque cylindre 11 que la vis pédiculaire 80 peut être associée à la sphère 40 correspondante.

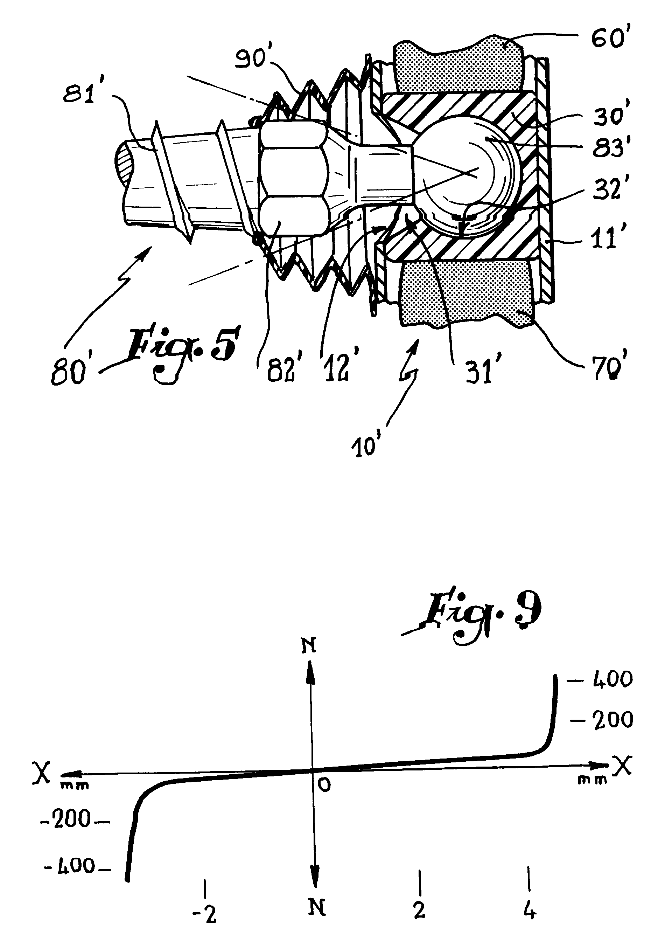

On a montré en fig. 5 une seconde variante de l'amortisseur afin qu'il puisse être monté sur des vis pédiculaire 80' particulières.

L'amortisseur comprend deux éléments identiques 10' pourvus chacun d'un cylindre 11' ouvert à ses deux extrémités et dont l'une d'entre elles est fermée par un fond non représenté, mais identique à celui référencé 20 (fig. 4).

Dans la partie médiane du cylindre 11' est percé un trou transversal 12' non débouchant. Dans le cylindre 11' est engagé un manchon tubulaire 30' qui est réalisé en une matière plastique appropriée telle que le polyéthylène. Le manchon 30' comporte un alésage 31' à profil conique dont la base la plus large est tournée vers l'extérieur dudit manchon. L'alésage 31' débouche dans une cavité 32' en forme de sphère.

De part et d'autre du manchon 30' sont prévus deux blocs élastiques 60' et 70' identiques à ceux décrits dans l'amortisseur de la fig. 4. La vis pédiculaire 80' comporte une partie filetée 81' qui permet son ancrage dans l'apophyse d'une vertébre. La base la plus large de la partie filetée 81' est solidaire d'un profil hexagonal 82' qui est prolongé suivant l'axe principal de la vis 80' par une tête sphérique 83' dont le rayon correspond à celui de la cavité 32' du manchon 30' .

Le profil hexagonal 82' permet à l'opérateur de visser la vis pédiculaire 80' dans l'apophyse de la vertèbre à stabiliser. Une fois la vis pédiculaire 80' ancrée dans l'apophyse, sa tête sphérique 83' est engagée à force dans la cavité 32' du manchon 30' . On observe que préalablement à la mise en place de l'amortisseur sur la tête sphérique 83' de la vis pédiculaire 80', il est prévu de placer autour de celle-ci un soufflet 90' qui entoure la partie hexagonale 82' et qui vient prendre appui sur la face extérieure du cylindre 11' . Le soufflet 90' est comprimé au moment du montage de manière que l'alésage 12' ne soit jamais en contact avec les parties telles que du sang ou de la chair qui risquerait de bloquer les déplacements angulaires de l'amortisseur.

Le fonctionnement est le suivant : deux vis pédiculaires 6 ou 80 ou 80' étant ancrées dans deux vertèbres voisines dont le disque commun est endommagé, on obtient grâce à l'amortisseur suivant l'invention un amortissement du mouvement relatif des deux vertèbres en extension et en flexion.

En effet, si les deux vis pédiculaires 6 ou 80 ou 80' ont tendance à s'écarter, ce qui provoque un écartement des deux sphères 5a ou 40 ou 83', le bloc 7 de l'élément 1 se trouvant dans la partie inférieure de l'amortisseur ou les deux blocs 60 ou 60' sont mis en compression et se déforment. Mais la déformation est contrecarrée par la rigidité des parois de chaque cylindre 1a ou 11 ou 11' de sorte que le bloc élastique s'oppose au déplacement du manchon 4 ou 30 ou 30' avec une réaction croissante. Lorsque le manchon 4 ou 30 ou 30' occupe tout le volume de la chambre dans laquelle il est disposé, la réaction développée par ce bloc devient exponentielle et à la limite forme une butée interdisant tout déplacement subséquent du manchon 4 ou 30 ou 30' . En conséquence, le déplacement intervertébral est limité.

Il en va de même lorsque les deux sphères 5a ou 40 ou 83' ont tendance à se rapprocher. A ce moment là, il se produit le même phénomène que celui décrit plus haut, mais qui est compensé grâce au bloc 7 de l'élément 1 se trouvant dans la partie supérieure de l'amortisseur ou aux deux blocs 70 ou 70' .

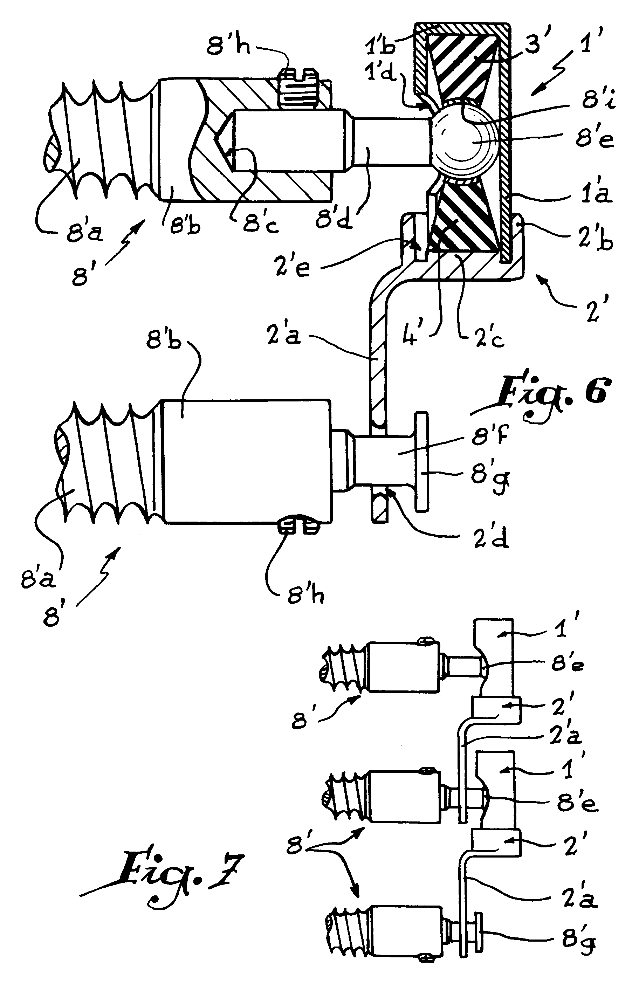

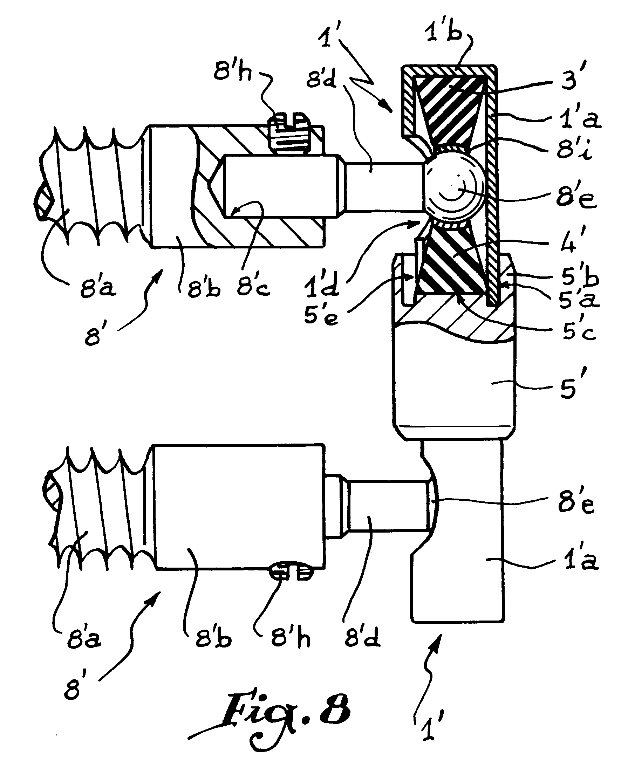

On a représenté en fig. 6 et 7 une troisième variante de l'amortisseur suivant l'invention qui comprend un élément 1' monté sur des vis pédiculaires 8' préalablement ancrées dans les vertèbres.

L'élément 1' comprend un cylindre creux 1'a dont l'une des extémités est fermée par un fond 1'b, tandis que l'autre qui est prévue ouverte est destinée à recevoir un disque 2' solidaire d'une patte de fixation 2'a qui est percée à son extrémité libre d'un trou 2'd. La patte 2'a est prévue décalée latéralement par rapport au milieu du disque 2' pour être dans un plan parallèle à celui passant par l'axe vertical de l'élément 1' .

Le cylindre 1'a est percé d'une lumière ou encoche longitudinale 1'd qui débouche du côté de l'extrémité qui doit recevoir le disque 2' . Ce dernier comprend une jupe 2'b s'étendant verticalement de manière à guider axialement le cylindre creux 1'a. Entre les parois de la jupe 2'b est prévue une surface d'appui 2'c de forme cylindrique et de diamètre sensiblement équivalent à celui interne du cylindre 1'a. La jupe 2'b et la surface d'appui 2'c délimitent une encoche 2'e dans laquelle est introduite l'extrémité ouverte du cylindre creux 1'a en vue de coopérer avec le disque 2' .

Les vis pédiculaires 8' ancrées dans les vertèbres de la colonne vertébrale comportent une partie filetée auto-taraudante 8'a et une tête cylindrique 8'b. Cette dernière est percée en son milieu d'un trou borgne 8'c permettant la mise en place soit d'une tige cylindrique 8'd munie à l'une de ses extrémités d'une tête sphérique 8'e, soit d'un doigt cylindrique 8'f comportant à l'une de ses extrémités une butée 8'g. La tige cylindrique 8'd et le doigt 8'f sont maintenus à l'intérieur de chaque trou borgne 8'c correspondant par l'intermédiaire d'une vis de pression 8'h ou tout autre moyen analogue.

Sur le fond 1'b de l'élément 1' est placé un corps élastique 3' tel qu'un bloc de caoutchouc naturel ou synthétique. La tige 8'd est introduite par l'intermédiaire de l'encoche 1'd à l'intérieur du cylindre creux 1'a de manière que sa tête sphérique 8'e vienne en appui contre le corps élastique 3' . Préalablement à la mise en place de la tige 8'd, il est placé autour de la tête sphérique 8'e un anneau 8'i dont les faces intérieures et extérieures affectent un profil en portion de sphère de même diamètre que celui de ladite tête 8'e. L'anneau 8'i est réalisé en une matière telle que du métal ou du TEFLON. Un autre corps élastique 4' identique à celui référencé 3' est placé à l'intérieur du cylindre creux 1'a pour venir en appui contre l'anneau 8'i solidaire de la spère 8'e. L'extrémité opposée à celle 1'b du cylindre creux 1'a est ensuite fermée par le disque 2' pour que sa surface 2'c soit en appui contre le corps élastique 4' .

Il peut être prévu à la place de l'anneau 8'i une cupule de même profil qui est placée de part et d'autre de la tête sphérique 8'e afin que cette dernière ne prenne pas appui directement sur les corps élastiques.

On note que la tige cylindrique 8'd comportant la tête sphérique 8'e associée à son anneau 8'i est montée à pivotement entre deux corps élastique 3' et 4' afin d'amortir ses déplacements lorsque les vertèbres sont soumises à des efforts de tractions/compressions.

Les corps élastiques 3' et 4' peuvent par exemple présenter un profil conique dont la base la moins large est en appui respectivement contre le fond 1'b de l'élément 1' et la surface 2'c du disque 2' .

Suivant le nombre de disques à soulager le chirurgien procèdera à des montages différents des éléments 1' comme ceux par exemple représentés en fig. 6 et 7.

En effet lorsqu'un seul disque doit être soulagé le chirurgien fixera préalablement deux vis 8' dans chacune des vertèbres se trouvant au-dessus et en-dessous dudit disque. Il introduit dans la première vis 8', c'est-à-dire celle se trouvant au-dessus du disque à soulager une tige 8'd solidaire de l'élément 1' de manière que l'extrémité libre de la patte 2'a du disque 2' et plus particulièrement son trou 2'd coopère avec un certain jeu avec le doigt 8'f qui est placé à l'intérieur de la seconde vis 8' disposée dans la vertèbre se trouvant en dessous du disque (fig. 6). Ainsi ce montage particulier permettra lors du déplacement des vertèbres de soulager et d'amortir les efforts de compression et de traction soumis au disque.

Par contre, lorsque plusieurs disques sont à soulager le chirurgien procède suivant l'assemblage représenté en fig. 7. Il est possible de monter plusieurs éléments 1' les uns au-dessus des autres de manière que chaque patte 2'a des disques 2' coopère avec la tige 8'd de l'élément 1' se trouvant directement en-dessous. Le premier élément 1' de l'assemblage prend appui sur une vis pédiculaire 8' associée à un doigt 8'f solidaire de la butée 8'g comme cela est préalablement décrit en fig. 6. Cet assemblage peut être répété pour plusieurs étages à soulager.

En fig. 8 on a montré un assemblage particulier de deux éléments 1' identiques et décrits précédemment. Le montage est prévu pour soulager par exemple un disque compris entre deux vertèbres de la colonne vertébrale. Les deux éléments 1' sont reliés par l'intermédiaire d'un manchon cylindrique 5' . Le manchon 5' est prévu pour remplacer le disque 2' décrit précédemment. Le manchon 5' comprend à chaque extrémité un trou borgne 5'a dont le diamètre interne est équivalent à celui externe des cylindriques creux 1'a de chaque élément 1' à réunir. Dans le fond de chaque trou borgne 5'a est prévue une surface d'appui 5'c identique à celle référencée 2'c et de diamètre équivalent à celui interne du cylindre 1'a de manière à déterminer une encoche cylindrique 5'e pour la fixation des éléments 1' . Le trou borgne 5'a délimite sur sa périphérie une jupe 5'b qui guide axialement le cylindre 1'a et le maintient verticalement. La surface d'appui 5'c est prévue pour recevoir l'une des extrémités des corps élastiques 3' ou 4' de chaque élément 1' a réunir. Cet assemblage permet de la même manière que précédemment d'amortir les efforts de traction et compression pour soulager les disques endommagées.

Bien évidemment un soufflet non représenté mais identique à ceux référencés 8, 90' entoure la tige 8'd solidaire des vis pédiculaires 8' pour protéger la partie interne des éléments 1' .

En fig. 9, on a représenté la courbe qui illustre la variation de la force de résistance de l'amortisseur suivant la présente invention. La partie gauche de la courbe correspond à un effort de compression appliqué sur l'amortisseur et qui présente en fait un déplacement négatif illustré à partir de l'origine. La réaction N croît pour la majorité du déplacement de manière relativement faible, c'est-à-dire qu'elle est approximativement tangente à l'axe des X représentant le déplacement. Elle devient ensuite exponentielle pour finir asymptotique à une droite non représentée mais parallèle à l'axe des forces N.

Si, au contraire, le déplacement de l'amortisseur est positif (cas d'une traction) la courbe illustrant la résistance antagoniste de l'amortisseur est symétrique par rapport à celle correspondant à une compression, cette partie de la courbe devenant asymptotique par rapport à une droite non représentée, mais également parallèle à l'axe des forces positives N.

On obtient donc une régulation du mouvement de flexion-extension entre deux vertèbres en développant une courbe des forces N et des déplacements X exponentiels dans les deux directions, ce qui correspond à l'allure physiologique du mouvement discal.

Lors du fonctionnement des différentes prothèses décrites précédemment, on s'aperçoit qu'elles définissent une amplitude de flexion-extension dont les limites correspondent aux zones verticales de la courbe représentée en fig. 9. Ces zones verticales peuvent être modifiées pour contrôler le secteur de mobilité en flexion-extension.

Bien entendu, la courbe est totalement symétrique si les corps élastiques 7 ou 60, 70 ou 60', 70' ou 3', 4' présentent les mêmes caractéristiques de volume et de flexibilité et que les chambres qui les reçoivent sont de même volume. Par contre, on peut faire varier et les caractéristiques des blocs élastiques et les volumes des chambres les recevant de manière à obtenir des efforts résistants différents à la compression ou à la traction.

On note que les amortisseurs décrits ci-dessus peuvent être superposés par rapport au rachis grâce à des vis pédiculaires comportant plusieurs têtes sphériques qui sont alignées suivant l'axe principal de ladite vis. On constate qu'il existe une liaison entre chaque vertèbre, qui est totalement stabilisée que ce soit en traction ou en compression par l'intermédiaire de l'amortisseur. On constate que les corps élastiques de chaque prothèse décrite précédemment peuvent être associés chacun à un ressort pour améliorer leur effet d'amortisseur, tandis qu'ils peuvent présenter un profil extérieur de forme quelconque.

On remarque que les amortisseurs décrits créent des dispositifs de stabilisation inter-vertébrale, toutefois d'autres applications médicales peuvent être envisagées. Ces amortisseurs peuvent par exemple être utilisés en combinaison avec un système prothétique quel qu'il soit comme par exemple un dispositif d'ostéosynthèse pour créer une combinaison d'une zone rachidienne fixe dont les extrémités sont mécaniquement assistés et souples.

## Revendications

1. Amortisseur du genre comprenant un corps élastique dont le volume est inférieur à celui de la chambre déterminée par la position à l'état libre du piston par rapport au fond du cylindre, caractérisé en ce qu'il est constitué d'éléments (1 ou 1' ou 10 ou 10') comportant des moyens pour la régulation du mouvement de flexion-extension entre deux vertèbres de manière à se rapprocher le plus possible de l'allure physiologique d'un mouvement discal.

2. Amortisseur suivant la revendication 1, caractérisé en ce que chaque élément (1) comprend un cylindre creux (1a) percé d'une lumière débouchante (1d) et dont l'une des extrémités est fermée par un fond (1b) solidaire sur sa face externe d'un tenon (1c) tandis que l'autre extrémité reçoit un disque (2) qui permet par l'intermédiaire d'une bague d'assemblage (3) la liaison avec un autre élément (1), un manchon (4) placé dans le cylindre (1) et dont l'une des extrémités est fermée par un fond (4a) alors que l'autre reçoit une face du disque (2) sur laquelle est prévu un bossage (2a), une encoche (4b) étant ménagée sur la périphérie du manchon (4) et une vis 5 dont la tête sphérique (5a) est montée à pivotement entre le disque (2) et le fond (4a) et un corps élastique (7) placé entre le manchon (4) et le fond (2b) du cylindre (1) de manière à amortir les déplacements de celui-ci sous l'effet des déplacements de la vis.

3. Amortisseur suivant la revendication 1, caractérisé en ce que chaque élément (10 ou 10') comprend un cylindre creux (11 ou 11') dont l'une des extrémités est fermée par un fond (20) tandis que l'autre extrémité comporte une bague d'assemblage (50) qui permet la liaison avec un autre cylindre (11 ou 11'), la partie médiane des cylindres (11 ou 11') étant pourvue d'un trou transversal (12 ou 12') pour la mise en place d'un manchon tubulaire (30 ou 30') qui est prévu pour recevoir à force une sphère (40, 83') solidaire d'une vis pédiculaire (80 ou 80'), et en ce qu'entre chaque manchon (30 ou 30') et le fond (20) est placé un corps élastique (60 ou 60') alors qu'un autre corps (70 ou 70') est introduit entre chaque manchon et la bague d'assemblage (50).

4. Amortisseur suivant la revendication 1, caractérisé en ce que chaque élément (1') comprend un cylindre creux (1'a) percé d'une lumière débouchante (1'd) et dont l'une des extrémités est fermée par un fond (1'b) tandis que l'autre extrémité reçoit un disque (2') solidaire d'une patte d'assemblage (2'a) permettant la liaison soit avec un autre élément (1') soit avec un doigt (8'f) solidaire d'une vis pédiculaire (8'), et une tige cylindrique (8'd) dont la tête sphérique (8'e) associée à un anneau (8'i) est monté à pivotement entre deux corps élastiques (3' et 4') respectivement placés en appui contre le fond (1'd) et le disque (2') de l'élément (1').

5. Amortisseur suivant la revendication 2, caractérisé en ce que le disque (2) comporte sur la face opposée à celle comprenant le bossage (2a) une saillie (2c) en forme de tenon permettant la liaison avec la bague d'assemblage (3).

6. Amortisseur suivant la revendication 2, caractérisé en ce que le bossage (2a) du disque (2) comporte une creusure (2b) pour recevoir la sphère (5a) de la vis (5).

7. Amortisseur suivant la revendication 2, caractérisé en ce que la bague d'assemblage (3) présente un profil cylindrique creux dont les extrémités parallèles (3a et 3b) sont pourvues d'une encoche (3c) qui coopèrent lors du montage de l'amortisseur avec les tenons (2c et 1c) de chaque élément (1).

8. Amortisseur suivant la revendication 7, caractérisé en ce que la bague (3) est rigide, ou souple, ou articulée.

9. Amortisseur suivant la revendication 2, caractérisé en ce que le manchon (4) présente dans sa partie interne une creusure (4c) dont le rayon est identique à celui de la creusure (2b) du disque (2) pour recevoir à rotation la sphère (5a) de la vis (5).

10. Amortisseur suivant la revendication 2, caractérisé en ce que la vis (5) présente à l'extrémité opposée à la sphère (5a) une partie filetée (5b) qui coopère avec un trou taraudé (6a) d'une vis pédiculaire (6).

11. Amortisseur suivant la revendication 10, caractérisé en ce que la vis (5) comporte entre la partie filetée (5b) et la sphère (5a) une portée (5c) et une partie cylindrique lisse (5d) qui est percée de part en part d'un trou transversal (5e) pour permettre le serrage de la vis (5) dans la vis pédiculaire (6).

12. Amortisseur suivant la revendication 3, caractérisé en ce que le manchon tubulaire (30) est pourvu d'un alésage (31) comportant dans sa partie médiane une creusure (32) de rayon identique à celui de la sphère (40).

13. Amortiseur suivant la revendication 3, caractérisé en ce que les deux cylindres (11 ou 11') sont assemblés au moyen d'une bague (50) qui est constituée par une virole cylindrique (51) dont les extrémités se vissent respectivement sur les deux bouts opposés et voisins des deux cylindres (11 ou 11').

14. Amortisseur suivant la revendication 3, caractérisé en ce que la bague (50) est pourvue d'une paroi transversale médiane (52) d'épaisseur variable qui constitue une cloison séparatrice des deux cylindres (11 ou 11').

15. Amortisseur suivant la revendication 3, caractérisé en ce que chaque sphère (40) comporte un trou radial (41) dans lequel est introduite une extrémité cylindrique (82) de la vis pédiculaire (80) qui est immobilisée par l'intermédiaire d'une vis (90) dont la tête présente un diamètre plus grand que celui de ladite extrémité cylindrique.

16. Amortisseur suivant la revendication 3, caractérisé en ce que le manchon (30') comporte un alésage (31') qui débouche dans une cavité (32') en forme de sphère.

17. Amortisseur suivant la revendication 3, caractérisé en ce que la vis pédiculaire (80') comporte une partie filetée (81') qui est solidaire d'un profil hexagonal (82'), prolongée par une tête sphérique (83') dont le rayon est identique à celui de la cavité (32').

18. Amortisseur suivant la revendication 1, caractérisé en ce qu'un soufflet (8 ou 90') entoure les vis (5) ou les vis pédiculaires (80 ou 80') pour protéger le manchon (4 ou 30 ou 30').

19. Amortisseur suivant la revendication 4, caractérisé en ce que la patte (2'a) du disque (2') est décalée latéralement par rapport à l'axe principal de l'élément (1') pour se trouver dans un plan parallèle à ce dernier, tandis que son extrémité libre est percée d'un trou (2'b).

20. Amortisseur suivant la revendication 4, caractérisé en ce que le disque (2') est remplacé par un manchon (5') permettant de réunir deux éléments identiques (1').

21. Amortisseur suivant la revendication 20, caractérisé en ce que chaque extrémité du manchon (5') est percée d'un trou borgne (5'a) pour coopérer avec le cylindre creux (1'a) tandis que son fond comporte une surface d'appui (5'c) qui est en contact avec le corps élastique (4') de chaque élément (1') à réunir.

22. Amortisseur suivant la revendication 4, caractérisé en ce que la tige (8'd) et le doigt (8'f) sont respectivement retenus à l'intérieur d'un trou borgne (8'c) de la vis pédiculaire (8') correspondante par l'intermédiaire d'une vis de pression (8'h).

23. Amortisseur suivant la revendication 4, caractérisé en ce que l'anneau (8'i) est réalisé en une matière telle que du TEFLON ou du métal.

24. Amortisseur suivant la revendication 4, caractérisé en ce que les corps élastiques (3' et 4') sont de forme conique dont la base la moins large est tournée du côté de la tête sphérique (8'e) de la tige (8'd).

25. Amortisseur suivant l'une quelconque des revendications 2, 3 et 4, caractérisé en ce que les corps élastiques (7, 60 ou 60', 70 ou 70'', 3', 4') sont associés chacun avec un ressort pour améliorer leur effet et amortisseur.

26. Amortisseur suivant la revendication 1, caractérisé en ce que les éléments (1 ou 1' ou 10 ou 10') sont raccordés à un système d'osthéosynthèse rigide pour constituer une association comportant une zone rachidienne fixe dont les extrémités sont mécaniquement assistées et souples.
